# DEMANDE DE BREVET EUROPEEN

(11) **EP 0 870 755 A1**
(43) Date de publication de la demande: **14.10.1998**
(21) Numéro de dépôt: 98400818.5
(22) Date de dépôt: 06.04.1998
(51) Int. Cl.: C07C 209/18

(54) **Procédé de préparation des dinitroanilines 4-substituées**

(30) Priorité: 07.04.1997 FR 9704236; 02.07.1997 FR 9708360
(71) Demandeur: CFPI AGRO, 92233 Gennevilliers (FR)
(72) Inventeur: Schapira, Joseph, 75015 Paris (FR); Cheminaud, Jean-Claude, 95220 Herblay (FR); Gasse, Jean-Jacques, 27600 Gaillon (FR); Schanen, Vincent, 75019 Paris (FR); Rondot, Benoit, 92300 Levallois Perret (FR); Lemoine, Jean-Claude, 92290 Chatenay Malabry (FR)
(74) Mandataire: Koch, Gustave

(57) **Abrégé**

Procédé de préparation des dinitroanilines 4-substituées de formule 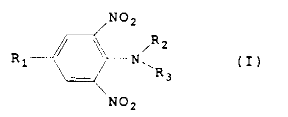 dans laquelle
- R₁ est choisi dans le groupe comprenant les radicaux tertbutyle, sulfonamido, trifluorométhyle, méthylsulfonyle, isopropyle,
- R₂ et R₃, qui sont identiques ou différents l'un de l'autre, représentent un atome d'hydrogène, un radical alkyle rectiligne ou ramifié, cycloalkyle, halogénoalkyle ou alkényle comprenant moins de 6 atomes de carbone,
caractérisé par le fait qu'il comprend successivement:
- une étape de dinitration du phénol 4-substitué correspondant à la dinitroaniline 4-substituée recherchée, cette étape de dinitration étant effectuée au sein d'un milieu réactionnel comprenant un faible excès d'agent nitrant, une quantité suffisante de protons et un catalyseur choisi dans le groupe comprenant les sels solubles des métaux de transition des colonnes IV à XII de la classification périodique, de préférence les sels solubles des ions Fe_{III,} Fe_{II}, Zn_{II} et Cu_{II},
- une étape d'O-alkylation du phénol 4-substitué dinitré obtenu à l'étape précédente, à l'aide d'un agent alkylant, et
- une étape d'amination du phénoxy-éther dinitré 4-substitué obtenu à l'étape précédente par mise en oeuvre d'une amine primaire ou secondaire.

## Description

L'invention a pour objet un procédé de préparation des dinitroanilines 4-substituées.

Les dinitroanilines en question, qui sont des herbicides connus, agissent essentiellement comme inhibiteurs de la division cellulaire.

Elles sont représentées par la formule 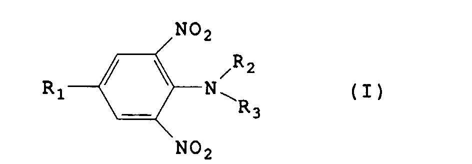 dans laquelle
- R₁ est choisi dans le groupe comprenant les radicaux tertbutyle, sulfonamido, trifluorométhyle, méthylsulfonyle, isopropyle,
- R₂ et R₃, qui sont identiques ou différents l'un de l'autre, représentent un atome d'hydrogène, un radical alkyle rectiligne ou ramifié, cycloalkyle, halogénoalkyle ou alkényle comprenant moins de 6 atomes de carbone.

Des dinitroanilines particulièrement intéressantes sont la butraline, la trifluraline et l'oryzaline respectivement représentées par les formules:

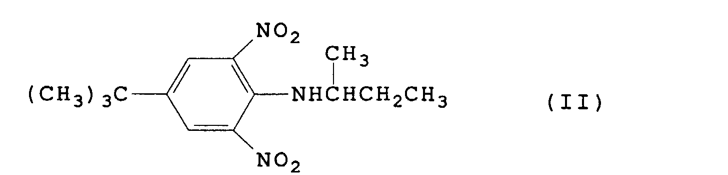

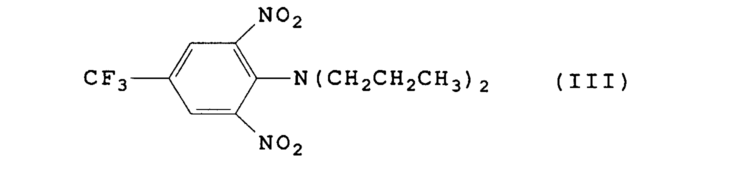

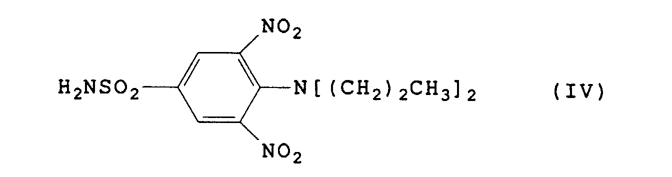

Il a déjà été proposé, par le brevet FR-A-70 41719, de préparer la butraline en procédant successivement:
- à la dinitration du 4-tertbutyl-phénol en utilisant l'acide nitrique en milieu acétique,
- à la chloration du 2,6-dinitro-4-tertbutyl-phénol obtenu à l'étape précédente en ayant recours au chlorure de thionyle ou à l'oxychlorure de phosphore en présence d'un amide tel que le formamide ou le diméthylformamide (DMF),
- à la condensation du 4-tertbutyl-2,6-dinitro-chlorobenzène formé à l'étape précédente avec deux moles de sec-butylamine.

La dinitroaniline ainsi obtenue doit être recristallisée en milieu alcoolique.

Si cette voie de synthèse est remarquable sur le plan théorique par sa simplicité due au nombre limité de stades intermédiaires, sa mise en oeuvre se révèle difficile et une purification de chacun des intermédiaires est indispensable pour obtenir un produit de qualité.

De plus, la nitration conduit à un effluent nitroacétique qui est extrêmement difficile et coûteux à traiter et bien sûr encore plus à recycler dans le procédé.

Par ailleurs, la chloration consomme des excès de chlorure de thionyle et/ou d'oxychlorure de phosphore qui sont des produits chers, ce qui rend ce procédé économiquement non compétitif.

Enfin, il y a des risques de formation de dérivés détonants au moment de la nitration.

D'autres procédés connus utilisent, comme matières premières, des alkylchlorobenzènes ou des alkylanilines .

Ces procédés connus présentent également des inconvénients importants.

Ainsi,
- la matière première constituée par les alkylchlorobenzènes n'est pas disponible commercialement, ce qui nécessite une étape de chloration supplémentaire et
- lorsque la matière première est une alkylaniline, il y a formation de N-nitrosamines en quantité importante.

L'invention a donc pour but, surtout, de remédier aux inconvénients de l'art antérieur et de fournir un procédé de préparation des dinitroanilines 4-substituées qui réponde mieux que ceux qui existent déjà aux divers desiderata de la pratique.

Et la Société Demanderesse a le mérite d'avoir trouvé, à l'issue de recherches approfondies, que ce but pouvait être atteint par la mise en oeuvre d'un procédé caractérisé par le fait qu'il comprend successivement:
- une étape de dinitration du phénol 4-substitué correspondant à la dinitroaniline 4-substituée recherchée, cette étape de dinitration étant effectuée au sein d'un milieu réactionnel comprenant un faible excès d'agent nitrant, une quantité suffisante de protons et un catalyseur choisi dans le groupe comprenant les sels solubles des métaux de transition des colonnes IV à XII de la classification périodique, de préférence les sels solubles des ions Fe_{III}, Fe_{II}, Zn_{II} et Cu_{II},
- une étape d'O-alkylation du phénol 4-substitué dinitré obtenu à l'étape précédente, à l'aide d'un agent alkylant choisi dans le groupe comprenant, d'une part, les mono-halogénures d'alkyle linéaires ou ramifiés ayant au moins 3 atomes de carbone et pouvant comprendre un cycle saturé ou au moins une insaturation, d'autre part, les polyhalogénures d'alkyle linéaires ou ramifiés ayant au moins 2 atomes de carbone et pouvant comprendre un cycle saturé ou au moins une insaturation, d'autre part encore les polyalcoxy-haloalkyléthers représentés par la formule (RO)ₙR'X dans laquelle X représente un atome d'hydrogène, de chlore, de brome ou d'iode et R et R', indépendamment l'un de l'autre, un radical méthyle, éthyle ou propyle, n étant tel que le nombre d'atomes de carbone de l'agent alkylant est ≥2, notamment les chlorométhyl polyméthoxy éthers et les chlorométhyl-polyéthoxy éthers, les oxydes d'éthylène et de propylène et, d'autre part encore, les composés de structure répondant à la formule 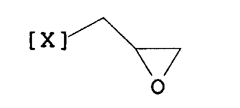 qui représente des épihalohydrines lorsque X représente Cl ou Br, le glycidol lorsque X représente OH, les éthers de glycidyle lorsque X représente OR, R étant une chaîne aliphatique d'au plus 12 atomes de carbone, ou un oxiranne lorsque X est une chaîne aliphatique R de 1 à 12 atomes de carbone,

- une étape d'amination du phénoxy-éther dinitré 4-substitué obtenu à l'étape précédente par mise en oeuvre d'une amine primaire ou secondaire.

Selon un mode de réalisation avantageux du susdit procédé, le catalyseur mis en oeuvre pendant l'étape de dinitration est choisi dans le groupe comprenant les chlorures ferriques et ferreux, les chlorures de zinc et cuivrique, les nitrates ferriques, zinciques et cuivriques ainsi que les sulfates ferriques ferreux, zinciques et cuivriques.

La proportion de catalyseur par rapport au phénol est de 1 à 50‰, de préférence de 5 à 30‰.

L'agent nitrant est choisi dans le groupe comprenant l'acide nitrique et les nitrates alcalins et alcalino-terreux solubles dans l'eau.

L'excès d'agent nitrant mis en oeuvre pour l'étape de dinitration est avantageusement d'environ 10% à 30% en poids.

La quantité de protons qui doit être présente dans le milieu nitrant est de 2 à 7, de préférence de 2,4 à 6 et, plus préférentiellement encore, de 2,6 à 2,9 équivalents par rapport au phénol et peut être apportée par un acide appelé co-acide et constitué par un acide minéral choisi dans le groupe comprenant les acides chlorhydrique, bromhydrique, sulfurique et phosphorique, ou par un acide organique choisi dans le groupe comprenant les acides carboxyliques inférieurs dont notamment les acides acétique, propionique et oxalique.

L'étape de nitration peut être réalisée en milieu nitrique homogène ou en présence d'un solvant inerte.

Le solvant inerte est avantageusement choisi dans le groupe comprenant les essences aliphatiques dont notamment l'hexane, l'heptane, l'octane, le nonane et le dodécane, les dérivés chlorés dont notamment le dichlorométhane, le 1,2-dichloroéthane, le chloroforme, le trichloréthylène, le tétrachloréthylène, et les dialkyl-éthers dont notamment les diéthyl- et dipropyl-éthers ainsi que les dibutyléthers.

La réaction est avantageusement conduite sous pression atmosphérique et à des températures de -20°C à +80°C.

Le dérivé 2,6-dinitré obtenu peut être séparé et purifié par les techniques conventionnelles bien connues de l'homme du métier.

Lorsque l'étape de nitration est effectuée sans solvant, le dérivé dinitré est précipité par dilution et glaçage du milieu réactionnel, puis séparé par filtration.

Au contraire, lorsque l'étape de nitration est effectuée au sein d'un solvant, le dérivé dinitré solubilisé dans la phase organique est isolé par décantation, l'agent nitrant contenu dans la phase aqueuse pouvant alors être recyclée au stade de la nitration après avoir été soumis au préalable à une concentration.

La pureté du dérivé 2,6-dinitro-phénol 4-substitué obtenu est généralement excellente et ne nécessite pas de purification ultérieure.

Lorsque le solvant est judicieusement choisi, il ne devient pas nécessaire d'isoler le dinitrophénol et la solution solvantée peut alors directement être soumise à l'étape suivante; c'est le cas par exemple lorsque le solvant appartient à la famille des solvants halogénés du groupe comprenant les chlorures de méthylène, le 1,2-dichloroéthane, le 1,2-dibromoéthane, le chloropropane, le chlorure d'isopropyle, le bromure d'isopropyle, les chlorures de butyle, le 1,2-dichloropropane et le 1,2-dibromopropane.

Le rendement de l'étape de dinitration est d'au moins 92% et peut atteindre 99%.

Selon un autre mode de réalisation avantageux du procédé conforme à l'invention, l'agent alkylant mis en oeuvre pendant l'étape d'O-alkylation est soit un monohalogénure d'alkyle représenté par la formule CₙH₂ₙ₋₁X dans laquelle X est un atome de chlore, brome ou iode et n est ≥ 3, soit un polyhalogénure d'alkyle représenté par la formule CₙH₂ₙ₋ₘX₂₊ₘ dans laquelle X est un atome de chlore, brome ou iode et n ≥ 2 avec O ≤ m ≤ 2n.

L'agent alkylant peut être choisi dans le groupe comprenant,
- en tant que monohalogénures d'alkyle, les halogénures de n-propyle, d'isopropyle, de n-butyle, de sec-butyle, d'isobutyle, de tertbutyle, de n-pentyle, de sec-amyle, de 3-pentyle, de neo-pentyle, d'hexyle, d'isohexyle, de cyclohexyle, de cyclopropyle, de benzyle, d'allyle, et de polyalcoxyalkyle avec comme chaîne alcoxylée un radical méthoxy, éthoxy ou propanoxy,
- en tant que polyhalogénures d'alkyle, les dihaloéthanes, notamment le dichloroéthane et le dibromo-éthane, les dihalopropanes, notamment les 1,2- et 1,3-dichloropropanes.

Les conditions des réactions d'éthérification des dinitrophénols sont bien connues de l'homme du métier.

Généralement, ces réactions sont effectuées sur le phénol dinitré ou sur un phénate alcalin correspondant en milieu aqueux ou plus avantageusement en milieu biphasique par réaction de transfert de phase.

Le phénate alcalin peut être un dinitrophénate soit de sodium ou de potassium, soit d'un sel organique tel que l'ammonium, les tétralkyl-ammonium quaternaires et les tétralkylphosphonium quaternaires.

L'agent d'alkylation, en solution dans un solvant immiscible à l'eau, est mélangé avec une solution aqueuse du phénate alcalin en présence d'un catalyseur de transfert de phase qui peut être constitué par un halogénure d'ammonium ou un sel de phosphonium quaternaire.

Le solvant peut être choisi dans le groupe comprenant les solvants aromatiques, les essences aliphatiques et les cétones; de préférence, il est choisi dans le groupe comprenant le toluène, l'O-xylène, le m-xylène, le p-xylène, l'hexane, l'heptane, l'octane, le nonane, le dodécane, la méthyléthylcétone, la méthylisobutylcétone, la cyclohexanone et les mélanges de ces solvants.

Il est également possible d'utiliser comme solvant un excès d'agent alkylant.

Les catalyseurs préférés sont le chlorure de tétrabutylammonium, le bromure de tétrabutylammonium, le bromure de tétrabutyl-phosphonium, le chlorure de triéthyl-benzylammonium, le triméthyl-benzylammonium.

La réaction d'alkylation peut être conduite à une température de 15°C à 150°C, sous pression atmosphérique ou sous une pression pouvant aller jusqu'à 15 bars.

Les réactions par transfert de phase peuvent être pratiquées à des températures variant de -10°C à +50°C et à pression atmosphérique alors que les réactions mettant en oeuvre des halogénures d'alkyle inférieurs peuvent se faire sous une pression de plusieurs dizaines de bars.

Le phénoxyéther dinitré obtenu à l'issue de cette étape peut être isolé du milieu réactionnel par les techniques connues de l'homme du métier, telles que par exemple l'extraction par un solvant approprié ou encore la concentration suivie d'un fractionnement de la phase organique lorsque le procédé met en oeuvre une réaction de transfert de phase.

Lorsqu'un excès d'agent alkylant est utilisé comme solvant, le produit est isolé par solubilisation des sels solubles dans l'eau, un second lavage alcalin permettant de solubiliser le dinitrophénate qui n'a pas réagi; une contre-extraction en milieu acide du produit résultant de ce second lavage conduit alors à une solution de dinitrophénol qui peut être facilement recyclée dans une opération subséquente d'éthérification.

La phase organique isolée après le second lavage est soumise à une évaporation de l'excès d'agent alkylant qui peut être avantageusement réutilisé dans l'opération suivante.

Toutefois, lorsque le solvant est choisi dans le groupe comprenant le toluène, le xylène et les essences aliphatiques dont notamment l'heptane, l'octane, le nonane, le décane et le dodécane, le fractionnement n'est pas nécessaire et la solution organique obtenue à cette étape peut être soumise directement à l'étape d'amination.

Selon un autre mode de réalisation avantageux du procédé conforme à l'invention, l'étape d'amination est réalisée en faisant réagir, à la pression atmosphérique ou sous 3 à 5 atmosphères et à une température comprise entre 30°C et la température de reflux du milieu, le produit de l'étape d'O-alkylation avec un excès d'amine secondaire ou primaire choisie dans le groupe comprenant l'amino-2-butane, la N,N-diéthylamine, la N,N-dipropylamine, la N-éthyl,N-butylamine, la N-propyl-,N-méthyl-cyclopropylamine, la N-(2-chloroéthyl),N-propylamine, la N-propyl-,N-2-méthyl-2-propénylamine, N-éthyl-,N-2-méthyl-2-propénylamine au sein d'un solvant inerte choisi dans le groupe comprenant les alcools et les solvants aromatiques.

De façon tout à fait surprenante, le procédé conforme à l'invention permet d'atteindre des rendements très élevés, supérieurs à 96%, sans production de plus de 1 ppm de N-nitrosamines et sans risque de formation de produits détonants.

Les étapes successives du procédé selon l'invention apparaissent sur le schéma réactionnel suivant:

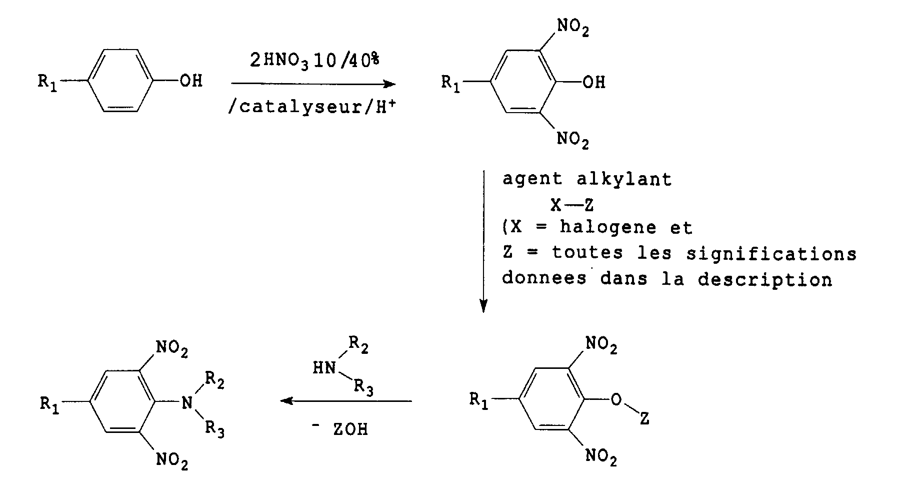

Lorsque l'agent alkylant est polyfonctionnel, c'est-à-dire constitué par exemple par un dihaloéthane, il est susceptible de réagir avec deux molécules de dinitrophénol et il se produit une polyéthérification illustrée par le schéma réactionnel:

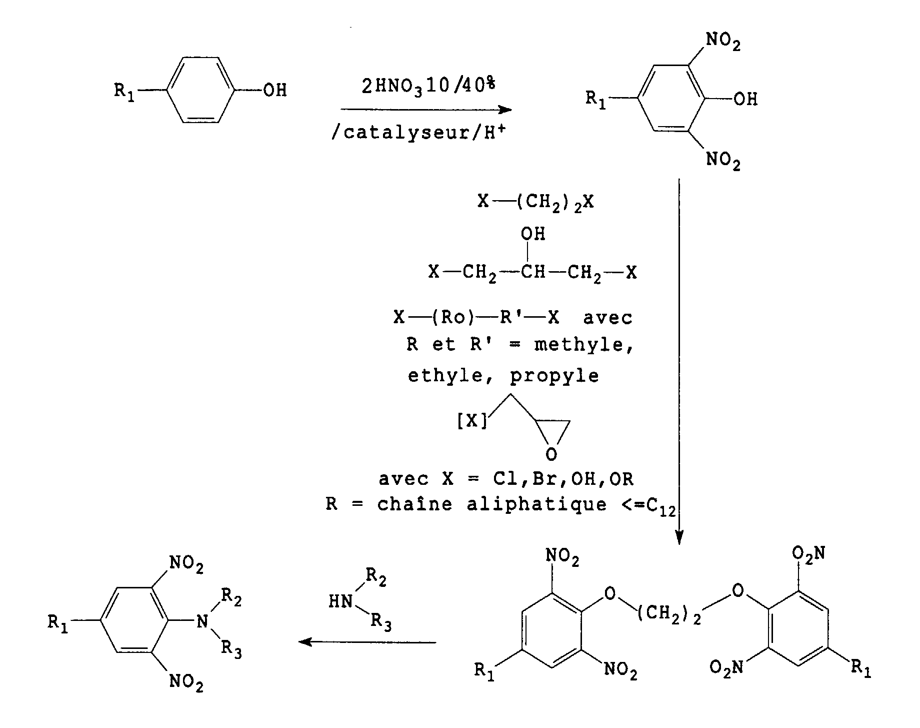

L'invention pourra être bien comprise à l'aide des exemples non limitatifs qui suivent et dont certains sont relatifs à des modes de réalisation avantageux.

Les exemples 1 à 15 illustrent l'étape de dinitration du phénol 4-substitué.

### EXEMPLE 1

Il s'agit d'un exemple comparatif dans lequel la dinitration est effectuée sans catalyseur.

On introduit en l'espace de 45 minutes et en maintenant la température à 20-25°C, une solution de 6,5 g (43,0 mmol) de 4-tertbutyl-phénol dans 26 g de chlorure de méthylène dans un milieu nitrant constitué d'un mélange de 17 g (108 mmol) d'acide nitrique à 40% et de 12 g de chlorure de méthylène.

Le milieu contient 108 mmol, soit 2,4 équivalents de protons par rapport au phénol.

L'excès d'agent nitrant est de 25%.

On laisse réagir pendant une heure à 20-35°C, puis pendant une autre heure à 40°C.

On sépare par décantation la phase organique de la phase aqueuse.

La phase aqueuse est contre-extraite avec 12,17 g de chlorure de méthylène.

Les phases organiques sont regroupées, séchées sur MgSO₄, puis concentrées à sec à l'aide d'un dispositif connu sous l'appellation "ROTAVAPOR".

On recueille 9,2 g d'un solide jaune brun constitué:
- de 72% de 2,6-dinitro-4-tertbutyl-phénol,
- de 22% de 2-nitro-4-tertbutyl-phénol,
- de 3,5% de 2,4-dinitro-phénol et
- de 2,3% de 4-nitrophénol.

Le rendement en produit recherché, à savoir en 2,6-dinitro-4-tertbutyl-phénol, est de 64,2%, donc médiocre.

### EXEMPLE 2

On introduit en l'espace de 10 minutes et en maintenant la température à 50°C, une solution de 4,5 g (30 mmol) de 4-tertbutyl-phénol dans 45 g de 1,2-dichloroéthane dans un mélange réactionnel nitrant constitué de 6,1 g (71 mmol) de nitrate de sodium dissous dans 32,2 g d'acide chlorhydrique à 22%; ce milieu nitrant contient 83 mg de chlorure de zinc.

L'excès d'agent nitrant est de 20%.

La quantité de protons présente dans le milieu est de 189 mmol, soit 6,3 équivalents par rapport au phénol.

On laisse réagir pendant une heure à 50-55°C, puis on laisse décanter. On contre-extrait la phase aqueuse avec 89 g de dichloréthane, puis on lave les phases organiques regroupées avec 50 g d'eau.

On sèche sur sulfate de magnésium, puis on évapore le solvant.

On obtient 6,88 g d'un composé constitué de:
- de 94,1% de 2,6-dinitro-4-tertbutyl-phénol,
- de 0,7% de 2-nitro-4-tertbutyl-phénol et
- de 3% de 2,4-dinitrophénol,
le rendement en produit recherché étant par conséquent de 95%.

### EXEMPLE 3

On introduit en l'espace de 5 minutes et en maintenant la température à 50°C, une solution de 4,5 g (30 mmol) de 4-tertbutyl-phénol dans 40 g de 1,2-dichloroéthane dans un mélange réactionnel nitrant constitué de 6,1 g (71 mmol) de nitrate de sodium dissous dans 22,7 g d'HCl à 17%; ce milieu nitrant contient 64 mg de chlorure zincique.

L'excès d'agent nitrant est de 20%.

La quantité de protons présente dans le milieu est de 103 mmol, soit 3,4 équivalents par rapport au phénol.

On laisse réagir pendant 30 minutes à 50°C, puis on maintient le milieu pendant 3 heures 30 minutes à 55°C.

On sépare les phases par décantation, on contre-extrait la phase aqueuse avec 40 g de dichloréthane, on regroupe les phases organiques et on les lave avec 20 g d'eau; elles sont ensuite séchées et le solvant est éliminé par évaporation.

On obtient 7,1 g d'un produit contenant 95,1% de 2,6-dinitro-4-tertbutyl-phénol dans lequel on ne retrouve pas de composé mononitré.

Le rendement en produit dinitré recherché est de 93,8%.

### EXEMPLE 4

On introduit en l'espace de 30 minutes et en maintenant la température à 50°C, une solution de 4,5 g (30 mmol) de 4-tertbutyl-phénol dans 40 g de 1,2-dichloroéthane dans un mélange réactionnel nitrant constitué de 22,6 g d'acide nitrique à 20% (72 mmol) et de 1,5 g d'acide chlorhydrique à 37% (15,2 mmol); le milieu nitrant contient 122 mg de chlorure ferrique hexahydrate.

L'excès d'agent nitrant est de 20%.

Le milieu contient une quantité de protons correspondant à 87,2 mmol, soit 2,9 équivalents par rapport au phénol.

On laisse réagir pendant 4 heures à 55°C, puis pendant 1 heure 30 minutes à 60°C.

On sépare les phases par décantation et on contre-extrait la phase aqueuse avec deux fois 20 g de 1,2-dichloréthane.

On regroupe les phases organiques que l'on lave avec 20 g d'eau distillée; ces phases sont ensuite séchées par évaporation.

On obtient 7,01 g d'un produit solide jaune contenant 96,5% de 2,6-dinitro-4-tertbutyl-phénol.

Le rendement en produit dinitré recherché est de 94%.

### EXEMPLE 5

On introduit en l'espace de 30 minutes et en maintenant la température à 50°C, une solution de 4,5 g (30 mmol) de 4-tertbutyl-phénol dans 40 g de dichloréthane dans un mélange réactionnel nitrant constitué de 23,5 g d'acide nitrique à 19,4% (72 mmol) contenant 120 mg de chlorure ferrique hexahydrate.

L'excès d'agent nitrant est de 20%.

Il n'y a pas d'autres protons que ceux apportés par l'acide nitrique, soit 72 mmol donc 2,4 équivalents par rapport au phénol.

On laisse réagir pendant 3 heures 30 minutes à 55°C, puis on maintient la température pendant 1 heure à 60°C.

On sépare les phases par décantation, puis on contre-extrait la phase aqueuse avec 40 g de dichloréthane.

On regroupe les phases organiques que l'on lave avec 20 g d'eau distillée; on les sèche puis on évapore le solvant.

On recueille 7,00 g d'un solide jaune contenant 90% de 2,6-dinitro-4-tertbutyl-phénol et 7,2% de 2-nitro-4-tertbutyl-phénol.

Le rendement en produit dinitré recherché est de 87,5%.

Cet exemple montre qu'en présence d'une quantité insuffisante de protons, la dinitration s'arrête à des taux de conversion insuffisants.

### EXEMPLE 6

Cet exemple montre que la quantité minimale de protons requise peut être apportée par un excès d'acide nitrique qui joue donc en plus de son rôle nitrant, le rôle du co-acide.

On introduit en l'espace de 30 minutes et en maintenant la température à 50°C, une solution de 4,5 g (30 mmol) de 4-tertbutyl-phénol dans 40 g de 1,2-dichloréthane dans un mélange réactionnel constitué de 29,9 g d'acide nitrique à 18,5% (soit 87,7 mmol), ce qui correspond au total des 72 mmol d'agent nitrant et des 15,2 mmol de co-acide de l'exemple 4); on ajoute 120 mg de chlorure ferrique hexahydrate.

L'excès d'agent nitrant est de 45%.

La proportion de protons est de 2,9 équivalents.

On laisse réagir pendant 2 heures à la température de 55°C. On sépare les phases par décantation.

On sépare la phase aqueuse que l'on extrait avec deux fois 20 g de 1,2-dichloréthane.

Les phases organiques sont rassemblées, lavées avec 20 g d'eau distillée et séchées par évaporation.

On recueille 7,09 g d'un solide orange constitué de
- 93,2% de 2,6-dinitro-4-tertbutyl-phénol et
- 7,1% de 2-nitro-4-tertbutyl-phénol.

Le rendement en produit dinitré recherché est de 92%.

### EXEMPLE 7

Cet exemple illustre la possibilité de recycler les effluents nitriques recueillis à l'issue d'une étape de dinitration donnée vers une étape de dinitration subséquente.

On introduit en l'espace de 30 minutes et en maintenant la température à 50°C, une solution de 9,0 g (60 mmol) de 4-tertbutyl-phénol dans 80 g de 1,2-dichloréthane dans un mélange réactionnel nitrant constitué de 32,5 g d'acide nitrique à 28% (144 mmol) et de 8,9 g d'acide chlorhydrique à 37% (90 mmol); ce milieu nitrant contient par ailleurs 310 mg de chlorure ferrique hexahydrate.

L'excès d'agent nitrant est de 20%.

La proportion de protons est de 3,9 équivalents.

On laisse réagir pendant 2 heures à la température de 55°C. On sépare les phases par décantation; la phase aqueuse est extraite avec 40 g de dichloréthane.

On récupère 34,6 g d'effluent nitrique constitué par une phase aqueuse comprenant:
- 3,1 g d'acide chlorhydrique et
- 1,44 g d'acide nitrique.

On regroupe les phases organiques, on les sèche sur sulfate de magnésium et on évapore à sec.

On recueille 14,18 g d'un solide jaune-orangé comportant 95,1% de 2,6-dinitro-4-tertbutyl-phénol, ce qui correspond à un rendement de 93,6%.

On établit un nouveau mélange réactionnel nitrant à partir de 20 g de l'effluent nitrique récupéré ci-dessus; cet effluent nitrique contient 13,3 mmol de HNO₃ et 49,9 mmol de HCl et il est reconcentré par l'addition de 3,71 g d'acide nitrique à 99% (58,3 mmol) et de 0,5 g d'acide chlorhydrique à 37% (5,1 mmol).

Dans ce nouveau milieu nitrant, on introduit en l'espace de 30 minutes et à une température de 50°C une solution de 4,5 g (30 mmol) de 4-tertbutyl-phénol dans 40 g de 1,2-dichloréthane.

L'excès en agent nitrant est de 20%.

La proportion de protons est de 4,2 équivalents.

On laisse réagir pendant 2 heures à 55°C.

Après décantation, on sépare la phase aqueuse que l'on extrait avec 40 g de 1,2-dichloréthane.

Les phases organiques sont regroupées, lavées avec 15 g d'eau distillée, puis séchées sur du sulfate de magnésium avant d'être évaporées à sec.

On recueille 7,1 g d'un solide jaune orangé contenant 96,4% de 2,6-dinitro-4-tertbutyl-phénol.

Le rendement en dérivé dinitré est de 95,1%.

### EXEMPLE 8

Cet exemple illustre l'étape de O-alkylation du dinitro-phénol 4-substitué.

Dans un ballon de 50 ml équipé d'un agitateur, d'un réfrigérant et d'un thermomètre, on charge 2,5 g de 2,6-dinitro-4-tertbutyl-phénate de tétrabutyl-ammonium obtenu par salification de 2,6-dinitro-4-tertbutyl-phénol, puis 50 g de 2-bromopropane.

On porte à 60 ±2°C durant 6 heures.

L'avancement de la réaction est suivi par chromatographie sur couche mince.

On refroidit ensuite à 25°C, puis on ajoute 30 g de 1,2-dichloréthane.

Le milieu réactionnel est lavé deux fois avec 30 g d'HCl 0,1N.

La phase organique, séparée après décantation, est lavée deux fois avec 10 g de soude 0,1M, puis avec 30 g d'eau distillée.

La phase organique est ensuite séchée sur sulfate de magnésium, puis évaporée sous vide.

On recueille 1,27 g d'un solide jaune-orangé constitué à 99,9% de 2-(2,6-dinitro-4-tertbutyl-phénoxy)-propane, obtenu avec un rendement de 86,7%.

Les phases aqueuses mères et de lavage sont contre-extraites à pH 1 avec 100 g de 1,2-dichloréthane.

Après séchage et évaporation du solvant, on recueille 0,15 g d'un solide orange constitué à 95% de 2,6-dinitro-4-tertbutyl-phénol n'ayant pas réagi et qui peut être recyclé vers une autre opération d'O-alkylation.

### EXEMPLE 9

Cet exemple illustre l'étape d'amination.

Dans un tricol de 25 ml équipé d'un agitateur, d'un réfrigérant et d'un thermomètre, on charge 1 g de 2-(2,6-dinitro-4-tertbutyl-phénoxy)propane à 99% de pureté, 5,2 g d'amino-2-butane et 8 g d'isopropanol.

On laisse réagir à 45°C pendant 2 heures, puis à 60°C pendant 7 heures.

En pratiquant une chromatographie sur couche mince d'une partie aliquote de milieu réactionnel, on détecte la fin de la réaction par disparition de l'éther.

On évapore le solvant et l'excès d'amine sous vide; le résidu obtenu est repris au dichlorométhane puis lavé avec 15 g d'HCl 0,1N, puis avec 15 g de soude à 0,1M, puis encore avec 20 g d'eau jusqu'à neutralité; il est ensuite séché puis évaporé à sec sous vide.

On obtient 1,02 g d'un solide jaune-orangé constitué à 99,1% (par CPG) en 4-tertbutyl-N(sec-butyl)-2,6-dinitro- benzènamine, ce qui correspond à un rendement de 97,5%.

### EXEMPLE 10

Cet exemple illustre les préparations de 3-(2,6-dinitro-4-tertbutyl-phénoxy)-1,2-oxopropane et d'hydroxy-poly(2-ol-propanoxy)-2,6-dinitro-4-tertbutyl-benzène.

Dans un tricol de 100 ml équipé d'un agitateur, d'un réfrigérant et d'un thermomètre, on charge 3,85 g (8 mmol) de 2,6-dinitro-4-tertbutyl-phénate de tétrabutylammonium obtenu par salification de 2,6-dinitro-4-tertbutyl-phénol, puis 25 g de toluène; on ajoute ensuite en 6 heures et à 100°C 11 g (82 mmol) d'épibromhydrine.

L'avancement de la réaction est suivi par chromatographie sur couche mince.

On refroidit à 25°C, on lave la phase aqueuse avec 10 g de toluène à pH 7, on regroupe les phases toluéniques et on les évapore à sec.

On recueille 3,6 g de polyéther brut qui est utilisé tel quel dans l'étape d'amination.

### EXEMPLE 11

Cet exemple illustre la formation de butraline à partir d'hydroxy-poly(2-ol-propanoxy)2,6-dinitro-4-tert-butyl-benzène.

Dans un tricol de 50 ml équipé d'un agitateur, d'un réfrigérant et d'un thermomètre, on charge 2,8 g du polyéther brut constitué par l'hydroxy-poly(2-ol-propanoxy)2,6-dinitro-4-tertbutyl-benzène préparé à l'exemple 10, puis 18,3 g (250 mmol) d'amino-2-butane; on porte la température à 65°C et on l'y maintient pendant 5 heures.

L'avancement de la réaction est suivi par chromatographie sur couche mince.

On distille sous vide l'excès d'amino-2-butane, on refroidit à 55°C, on charge 30 g de xylène et 15 g d'eau; lave la phase aqueuse avec 10 g de toluène, on regroupe les phases organiques toluéniques et on les évapore à sec.

On recueille 2,5 g d'un solide gommeux que l'on recristallise dans 5,5 g d'isopropanol.

On obtient 1,82 g d'un solide constitué à 82,5% (CPG) de 4-tertbutyl-N(sec-butyl)-2,6-dinitro-benzenamine.

Une chromatographie séparative du produit à 82,5% conduit à isoler une butraline à 98% de pureté.

### EXEMPLE 12

Cet exemple illustre l'étape de O-alkylation par un oxiranne d'un dinitrophénol 4-substitué.

Dans un réacteur de 100 ml à agitation magnétique muni d'un manomètre et d'une pastille de sécurité (40 bars), on charge 10 g de 2,6-dinitro-4-tertbutylphénol, 7,6 g de xylène et 0,04 g de pyridine. On refroidit à 10°C et on coule 3 g d'oxyde de propylène. On ferme le réacteur et on met sous 2-3 bars d'azote.

On porte à 120°C pendant 3 heures.

On refroidit à 30°C et on décompresse.

Après évaporation du xylène, on recueille 12,8 g de solide marron composé de 93,61% de 2-propanol-1-(2,6-dinitro-4-terbutylphénoxy) et de 4,1% de (2,6-dinitro-4-terbutylphénoxy)-propanoxy-propanol.

### EXEMPLE 13

Cet exemple illustre l'étape de O-alkylation par un oxiranne d'un dinitrophénol 4-substitué.

Dans un réacteur de 100 ml à agitation magnétique muni d'un manomètre et d'une pastille de sécurité (40 bars), on charge 35 g de 2,6-dinitro-4-tertbutylphénol, 35 g de xylène et 0,11 g de pyridine. On refroidit à 10°C et on coule 8,5 g d'oxyde d'éthylène. On ferme le réacteur et on met sous 2-3 bars d'azote.

On porte à 130°C pendant 5 heures.

On refroidit à 30°C et on décompresse.

Après évaporation du xylène, on recueille 41,46 g de solide orange composé de 92,65% d'éthanol-2-(2,6-dinitro-4-terbutylphénoxy) et de 3,01% de (2,6-dinitro-4-terbutyl-phénoxy)-éthoxy-éthanol.

### EXEMPLE 14

Cet exemple illustre l'étape d'amination du 1-(2,6-dinitro-4-terbutylphénoxy)2-propanol.

Dans un tricol de 50 ml équipé d'un agitateur magnétique, d'un réfrigérant (-10°C) et d'un thermomètre, on charge 11,3 g de 2-propanol-1-(2,6-dinitro-4-tertbutyl-phénoxy) à 95,93% de pureté et à 1,94% de produit de diaddition, 3,8 g d'amino-2-butane, 0,3 g de CaCl₂,6H₂0 et 34,2 g de xylène.

On maintient le mélange à reflux (50°C) pendant 8 heures.

En pratiquant une chromatographie sur couche mince d'une partie aliquote de milieu réactionnel, on détecte la fin de la réaction par disparition de l'éther.

On évapore le solvant et l'excès d'amine sous vide; le résidu obtenu est repris au dichlorométhane puis lavé avec 50 g d'eau; il est ensuite évaporé sous vide.

On obtient 10,2 g de solide orange constitué à 95,3% (déterminé par CPG) de N-(1-méthylpropyl)-2,6-dinitro-4-terbutyl-benzènamine.

### EXEMPLE 15

Cet exemple illustre l'étape d'amination du 1-(2,6-dinitro-4-terbutylphénoxy)2-éthanol.

Dans un tricol de 50 ml équipé d'un agitateur magnétique, d'un réfrigérant (-10°C) et d'un thermomètre, on charge 33,0 g d'éthanol-2-(2,6-dinitro-4-tertbutylphénoxy) à 95,0% de pureté et à 3,24% de (2,6-dinitro-4-terbutylphénoxy)éthoxyéthanol, 9,93 g d'amino-2-butane et 0,7 g de CaCl₂,6H₂O.

On maintient le mélange à reflux (65°C) pendant 2 heures.

En pratiquant une chromatographie sur couche mince d'une partie aliquote de milieu réactionnel, on détecte la fin de la réaction par disparition de l'éther.

On évapore ensuite le solvant et l'excès d'amine sous vide; le résidu obtenu est repris au cyclohexane puis lavé avec 25 g d'eau; il est ensuite évaporé sous vide.

On obtient 31,84 g de solide orange constitué à 96,6% (déterminé par CPG) de N-(1-méthylpropyl)-2,6-dinitro-4-terbutyl-benzènamine.

## Revendications

1. Procédé de préparation des dinitroanilines 4-substituées de formule 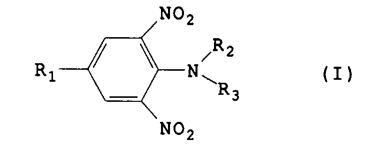 dans laquelle
- R₁ est choisi dans le groupe comprenant les radicaux tertbutyle, sulfonamido, trifluorométhyle, méthylsulfonyle, isopropyle,
- R₂ et R₃, qui sont identiques ou différents l'un de l'autre, représentent un atome d'hydrogène, un radical alkyle rectiligne ou ramifié, cycloalkyle, halogénoalkyle ou alkényle comprenant moins de 6 atomes de carbone, caractérisé par le fait qu'il comprend successivement:
- une étape de dinitration du phénol 4-substitué correspondant à la dinitroaniline 4-substituée recherchée, cette étape de dinitration étant effectuée au sein d'un milieu réactionnel comprenant un faible excès d'agent nitrant, une quantité suffisante de protons et un catalyseur choisi dans le groupe comprenant les sels solubles des métaux de transition des colonnes IV à XII de la classification périodique, de préférence les sels solubles des ions Fe_{III}, Fe_{II}, Zn_{II} et Cu_{II},
- une étape d'O-alkylation du phénol 4-substitué dinitré obtenu à l'étape précédente, à l'aide d'un agent alkylant choisi dans le groupe comprenant, d'une part, les mono-halogénures d'alkyle linéaires ou ramifiés ayant au moins 3 atomes de carbone et pouvant comprendre un cycle saturé ou au moins une insaturation, d'autre part, les polyhalogénures d'alkyle linéaires ou ramifiés ayant au moins 2 atomes de carbone et pouvant comprendre un cycle saturé ou au moins une insaturation, d'autre part encore les polyalcoxy-haloalkyléthers représentés par la formule (RO)ₙR'X dans laquelle X représente un atome d'hydrogène, de chlore, de brome ou d'iode et R et R', indépendamment l'un de l'autre, un radical méthyle, éthyle ou propyle, n étant tel que le nombre d'atomes de carbone de l'agent alkylant est ≥ 2, notamment les chlorométhyl polyméthoxy éthers et les chlorométhyl-poly-éthoxy éthers, les oxydes d'éthylène et de propylène et, d'autre part encore, les composés de structure répondant à la formule 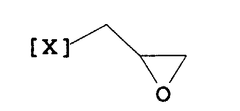 qui représente des épihalohydrines lorsque X représente Cl ou Br, le glycidol lorsque X représente OH, les éthers de glycidyle lorsque X représente OR, R étant une chaîne aliphatique d'au plus 12 atomes de carbone, ou un oxiranne lorsque X est une chaîne aliphatique R de 1 à 12 atomes de carbone,
- une étape d'amination du phénoxy-éther dinitré 4-substitué obtenu à l'étape précédente par mise en oeuvre d'une amine primaire ou secondaire.

2. Procédé selon la revendication 1, caractérisé par le fait que la proportion de catalyseur par rapport au phénol est de 1 à 50‰, de préférence de 5 à 30‰.

3. Procédé selon l'une des revendications 1 et 2, caractérisé par le fait que la quantité de protons présente dans le milieu nitrant est de 2 à 7, de préférence de 2,4 à 6 et, plus préférentiellement encore, de 2,6 à 2,9 équivalents par rapport au phénol et peut être apportée par un acide appelé co-acide et constitué par un acide minéral choisi dans le groupe comprenant les acides chlorhydrique, bromhydrique, sulfurique et phosphorique, ou par un acide organique choisi dans le groupe comprenant les acides carboxyliques inférieurs dont notamment les acides acétique, propionique et oxalique.

4. Procédé selon l'une des revendications 1 à 3, caractérisé par le fait que l'agent alkylant mis en oeuvre pendant l'étape d'O-alkylation est soit un monohalogénure d'alkyle représenté par la formule CₙH₂ₙ₋₁X dans laquelle X est un atome de chlore, brome ou iode et n est ≥ 3, soit un polyhalogénure d'alkyle représenté par la formule CₙH₂ₙ₋ₘX₂₊ₘ dans laquelle X est un atome de chlore, brome ou iode et n ≥ 2 avec O ≤ m < 2n.

5. Procédé selon la revendication 4, caractérisé par le fait que l'agent alkylant est choisi dans le groupe comprenant,
- en tant que monohalogénures d'alkyle, les halogénures de n-propyle, d'isopropyle, de n-butyle, de sec-butyle, d'isobutyle, de tertbutyle, de n-pentyle, de sec-amyle, de 3-pentyle, de neo-pentyle, d'hexyle, d'isohexyle, de cyclohexyle, de cyclopropyle, de benzyle, d'allyle, et de polyalcoxyalkyle avec comme chaîne alcoxylée un radical méthoxy, éthoxy ou propanoxy,
- en tant que polyhalogénures d'alkyle, les dihaloéthanes, notamment le dichloroéthane et le dibromo-éthane, les dihalopropanes, notamment les 1,2- et 1,3-dichloropropanes.

6. Procédé selon l'une des revendications 1 à 5, caractérisé par le fait que l'étape d'amination est réalisée en faisant réagir, à la pression atmosphérique ou sous 3 à 5 atmosphères et à une température comprise entre 30°C et la température de reflux du milieu, le produit de l'étape d'O-alkylation avec un excès d'amine secondaire ou primaire choisie dans le groupe comprenant l'amino-2-butane, la N,N-diéthylamine, la N,N-dipropylamine, la N-éthyl,N-butylamine, la N-propyl,N-méthyl-cyclopropylamine, la N-(2-chloro-éthyl),N-propylamine, la N-propyl,N-2-méthyl-2-propénylamine, la N-éthyl-,N-2-méthyl-2-propénylamine au sein d'un solvant inerte choisi dans le groupe comprenant les alcools et les solvants aromatiques.
